(19) Europäisches Patentamt
European Patent Office
Office européen des brevets

(11) Publication number : 0 498 771 A2

# (12) EUROPEAN PATENT APPLICATION

(21) Application number : 92810072.6

(51) Int. Cl.$^5$ : A61K 49/02

(22) Date of filing : 03.02.92

(30) Priority : 05.02.91 GB 9102450

(43) Date of publication of application :
12.08.92 Bulletin 92/33

(84) Designated Contracting States :
AT BE CH DE ES FR GB IT LI LU NL PT SE

(71) Applicant : SANDOZ LTD.
Lichtstrasse 35
CH-4002 Basel (CH)
(84) BE CH ES FR GB IT LI LU NL PT SE

(71) Applicant : SANDOZ-PATENT-GMBH
Humboldtstrasse 3
W-7850 Lörrach (DE)
(84) DE

(71) Applicant : SANDOZ-ERFINDUNGEN
Verwaltungsgesellschaft m.b.H.
Brunner Strasse 59
A-1235 Wien (AT)
(84) AT

(72) Inventor : Albert, Rainer
Rheinsprung 22/P
CH-4051 Basle (CH)

## (54) MSH peptide derivatives.

(57) Compounds of formula I

$$A[X - Y]_n \qquad I$$

wherein
A is a chelating group capable of complexing a detectable element,
n is 1 or 2
X is a direct bond or a spacer group, and
Y is a residue derived from a α-or β-MSH peptide bearing in the 10- or 11-position a substituted side chain group
A-X being linked to the terminal amino group of the peptide residue Y and the substitution on the side chain group in the 10- or 11-position being selected so that it does not significantly interfere with or impair the binding affinity of the peptide to the MSH receptors,
in free form or in a salt form are useful when labelled with a detectable element in in vivo diagnostic and therapeutic applications.

EP 0 498 771 A2

The present invention relates to MSH peptide derivatives, process for their production, pharmaceutical preparations containing them and their use as a pharmaceutical, e.g. as a radiopharmaceutical for imaging or therapy of melanomas.

In the last few years the presence of receptors for the melanocyte stimulating hormone has been demonstrated in melanomas. Prognosis for malignant melanomas is poor due to their tendency to early metastases. There thus exists a need for an agent with a chemically defined structure which could target melanomas and their metastases at an early stage.

The present invention provides new MSH peptides, particularly α-MSH peptides, which in labelled form are useful in in vivo diagnostic and therapeutic applications.

According to the invention, there is provided a compound of formula I

$$A[X - Y]_n \qquad (I)$$

wherein

A is a chelating group capable of complexing a detectable element,

n is 1 or 2

X is a direct bond or a spacer group, and

Y is a residue derived from a α- or β-MSH peptide bearing in the 10- or 11-position a substituted side chain group

A-X being linked to the terminal amino group of the peptide residue Y and the substitution on the side chain group in the 10- or 11-position being selected so that it does not significantly interfere with or impair the binding affinity of the peptide to MSH receptors.

The term MSH peptide includes the naturally occurring α-MSH as well as β-MSH and their analogues or derivatives.

The position of each amino-acid involved in the α-MSH peptide chain is numbered according to the accepted procedure beginning at position 1 for Ser on the amino end of the chain and ending with Val at position 13 at the other end of the chain as present in porcine α-MSH. In the following description, this same numbering system is applied to any peptide chain, even if it contains less than the 13 amino acid units present in the naturally occurring α-MSH, the position of each omitted amino-acid referring to the original numbering. Thus, in the compounds of the invention, the number 10 or 11 refers to the position 10 or 11, respectively, in the α-MSH peptide chain, independently whether the compounds contain 13 amino acid or fewer amino acid units. The same consideration applies to the numbering in β-MSH which comprises 18 amino acid units.

Compounds of formula I possess one chelating group capable of reacting with a detectable element, e.g. a radionuclide, a radioopaque element or a paramagnetic ion, to form a complex and further are capable of binding to MSH receptors, particularly α-MSH receptors, e.g. expressed or overexpressed by melanomas or metastases.

The chelating group is linked by a covalent bond to the amino group of the peptide, preferably by an amide or thioamide bond.

By derivatives or analogues as used herein is meant any polypeptide derived from that of the naturally occurring α- or β-MSH wherein one or more amino acid units have been omitted and/or replaced by one or more other amino acid radical(s) and/or wherein one or more functional groups have been replaced by one or more other functional groups and/or one or more groups have been replaced by one or several other isosteric groups. In general, the term covers all modified derivatives of α- or β-MSH peptide which e.g. have melanotropic activity or a binding affinity to MSH receptors.

Suitable spacer groups include e.g. a radical of formula (a)

$$-Z-R-CO- \qquad (a)$$

wherein

Z is a divalent group derived from a functional moiety capable of covalently reacting with a chelating agent, and

R is alkylene optionally interrupted by one or more heteroatoms or radicals selected from oxygen, sulfur, CO, -NHCO-, $N(C_{1-6}alkyl)$-CO-, -NH- and -$N(C_{1-6}alkyl)$-, hydroxy substituted alkylene, alkenylene,

$$-\underset{R_1}{\underset{|}{CH}}-,$$

or a radical of formula ($\alpha_1$)

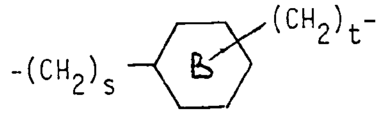

($\alpha_1$)

wherein each of s and t independently is 0, 1, 2 or 3, the ring B is optionally substituted phenyl or cyclohexylene and $R_1$ is a residue as attached in $C_\alpha$ of a natural or non natural $\alpha$-amino acid. $(CH_2)_t$- when present is preferably in para or meta.

Z may be for example a group which can form an ether, ester or amide bonding with the chelating agent. Z is preferably -CO- or -NH-.

Examples of substituents for ring B are e.g. hydroxy, halogen, $C_{1-3}$alkyl or $C_{1-3}$alkoxy.

$R_1$ may be H, $C_{1-6}$alkyl, benzyl, or -$CH_2$-naphthyl.

As mentioned above, the compounds of formula I bear either in position 10 or in position 11 an amino acid unit comprising a side chain group which is substituted. Preferably the substituted side chain group is a mono- or di-substituted side chain amino group. Examples of amino-acids bearing a side chain amino group include Orn, Lys, 2,6-diaminoheptanedioic acid (Dpm), 2,4-diaminobutyric acid (Dab) and 2,3-diaminopropionic acid (Dpr). Lys is particularly preferred.

Examples of $\alpha$-MSH analogues bearing a side chain amino group in position 10 or 11 are disclosed e.g. by T.K. Sawyer et al. in Proc. Natl. Acad. Sci. 77, 5754 (1980), F. Al-Obeidi et al., J. Med. Chem., 32, 174-179 (1989), F. Al-Obeidi et al. , J. Am. Chem. Soc., III, 3413-3417 (1989) and in EP-A-292,291. The contents of these publications including the specific compounds bearing a side chain amino group in position 10 or 11 are specifically incorporated herein by reference.

$\beta$-MSH analogues are disclosed e.g. by Geschwind and al. in J. Am Chem. Soc. 78, 4494-4495 (1956), in J. am. Chem. Soc. 79, 6394-6401 (1957), and by A. Eberle in The Melanotropins, edit. Karger, p. 20ff (1988). The contents of these publications including the specific compounds bearing a side chain amino in position 10 or 11 are specifically incorporated herein by reference.

Preferably n is 1.

Preferably X is a direct bond.

Y is preferably a residue derived from $\alpha$-MSH.

Preferred compounds of formula I are those derived from $\alpha$-MSH peptides bearing in position 10 or 11 a disubstituted side chain amino group; particularly preferred is $N^\varepsilon$-disubstituted Lys in position 10 or 11.

Preferred compounds of the invention are compounds of formula IA

$$A[X - Y_1]_n \qquad IA$$

wherein

A, X and n are as defined above, and

$Y_1$ is [$Nle^4$, D-$Phe^7$, mono- or disubstituted side chain $amino^{10}$ or $^{11}$]$\alpha$-MSH, particularly [$Nle^4$, $Asp^5$, D-$Phe^7$, mono- or disubstituted side chain $amino^{10}$ or $^{11}$]$\alpha$-MSH, more particularly [$Nle^4$, D-$Phe^7$, ($N^\varepsilon$ or $^\delta$-mono or -disubstituted Lys or Orn)$^{10}$ or $^{11}$]$\alpha$-MSH.

Preferably $Y_1$ is derived from 10- or 11-mono- or disubstituted [$Nle^4$, D-$Phe^7$, $Lys^{10}$, $Gly^{11}$] $\alpha$-$MSH_{1-13}$-$NH_2$; [$Nle^4$, D-$Phe^7$, $Lys^{10}$, $Glu^{11}$] $\alpha$-$MSH_{1-13}$-$NH_2$; [$Nle^4$, D-$Phe^7$, $Lys^{10}$, $Ser^{11}$] $\alpha$-$MSH_{1-13}$-$NH_2$; [$Nle^4$, D-$Phe^7$, $Lys^{10}$, $Nle^{11}$] $\alpha$-$MSH_{1-13}$-$NH_2$; [$Nle^4$, $Asp^5$, D-$Phe^7$, $Lys^{10}$, $Gly^{11}$] $\alpha$-$MSH_{1-13}$-$NH_2$; [$Nle^4$, D-$Phe^7$, $Lys^{10}$, $Gly^{11}$] $\alpha$-$MSH_{4-13}$-$NH_2$; [$Nle^4$, $Asp^5$, D-$Phe^7$, $Lys^{10}$, $Gly^{11}$] $\alpha$-$MSH_{4-13}$-$NH_2$; [$Nle^4$, D-$Phe^7$, $Lys^{10}$] $\alpha$-$MSH_{4-10}$-$NH_2$; [$Nle^4$, D-$Phe^7$, $Orn^{10}$] $\alpha$-$MSH_{4-10}$-$NH_2$; [$Nle^4$, $Asp^5$, D-$Phe^7$, $Orn^{10}$] $\alpha$-$MHS_{4-10}$-$NH_2$; [$Nle^4$, D-$Phe^7$, $Dab^{10}$] $\alpha$-$MSH_{4-10}$-$NH_2$; [$Nle^4$, $Asp^5$, D-$Phe^7$, $Dpr^{10}$] $\alpha$-$MHS_{4-10}$-$NH_2$; [$Nle^4$, $Asp^5$, D-$Phe^7$, $Lys^{10}$] $\alpha$-$MSH_{4-10}$-$NH_2$ and [$Nle^4$, $Asp^5$ D-$Phe^7$, $Dab^{10}$] $\alpha$-$MSH_{4-10}$-$NH_2$.

Examples of compounds of formula IA are those wherein $Y_1$ is

-Ser-Tyr-Ser-Met-Glu-His-DPhe-Arg-Trp-Gly-Lys*1 -Pro-Val-$NH_2$
-Ser-Tyr-Ser-Nle-Glu-His-DPhe-Arg-Trp-Lys*-Gly-Pro-Val-$NH_2$
-Ser-Tyr-Ser-Nle-Asp-His-DPhe-Arg-Trp-Lys*-Gly-Pro-Val-$NH_2$
-Nle-Glu-His-DPhe-Arg-Trp-Lys*-Gly-Pro-Val-$NH_2$
-Nle-Asp-His-DPhe-Arg-Trp-Lys*-Gly-Pro-Val-$NH_2$
-Nle-Asp-His-DPhe-Arg-Trp-Lys*-$NH_2$

*1 designating the side chain amino group which is mono or disubstituted according to the invention.

-Nle-Asp-His-DPhe-Arg-Trp-Dab*-$NH_2$
-Nle-Glu-His-DPhe-Arg-Trp-Lys*-$NH_2$
-Nle-Glu-His-DPhe-Arg-Trp-Orn*-$NH_2$
-Nle-Asp-His-DPhe-Arg-Trp-Orn*-$NH_2$
-Nle-Glu-His-DPhe-Arg-Trp-Dab*-$NH_2$
-Nle-Glu-His-DPhe-Arg-Trp-Dpr*-$NH_2$

When in the compounds of formula I the side chain group in position 10 or 11 is a side chain amino group, this group is preferably disubstituted with two identical substituents.

Preferably the substituent on the side chain amino group in position 10 or 11 of the compounds of formula I is a radical of formula (b)

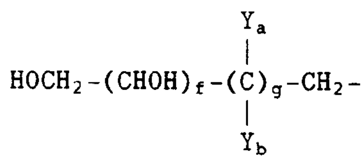 (b)

wherein one of $Y_a$ and $Y_b$ is hydrogen and the other is hydroxy or both are hydrogen, and each of f and g independently is 0 or 1.

Preferably f is 1 and g is 0.

Particular preferred compounds of formula I are those wherein Y is

-[$Nle^4$, $Asp^5$, D-$Phe^7$, ($N^\varepsilon$-bis-2,3-dihydroxypropyl-Lys)$^{10}$]$\alpha$-$MSH_{(4\text{-}10)}NH_2$

Suitable chelating groups A are physiologically acceptable chelating groups capable of complexing a detectable element. Preferably the chelating group A has substantially a hydrophilic character. Examples of chelating groups A include e.g. iminodicarboxylic groups, polyaminopolycarboxylic groups, e.g. those derived from non cyclic ligands e.g. ethylene diaminetetraacetic acid (EDTA), diethylene triamine pentaacetic acid (DTPA), N-hydroxyethyl-N,N′,N′-ethylene diaminetriacetic acid (HEDTA), ethylene glycol-0,0′-bis(2-aminoethyl)-N,N,N′,N′-tetraacetic acid (EGTA), N,N′-bis(hydroxybenzyl)ethylenediamine-N,N′-diacetic acid (HBED) and triethylenetetramine hexaacetic acid (TTHA), those derived from substituted EDTA or -DTPA, e.g. substituted by

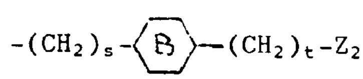

as defined below, those derived from macrocyclic ligands, e.g. 1,4,7,10-tetra-azacyclododecane-N,N′,N″,N‴-tetraacetic acid (DOTA), 1,4,8,11-tetraazacyclotetradecane-N,N′,N″,N‴-tetraacetic acid (TETA), 1,4,7,10-tetraazacyclotridecane-1,4,7,10-tetraacetic acid (TITRA), 1,4,8,11-tetraazacyclotetradecane (TETRA) or their C-functionalised derivatives thereof, for example such macrocyclic ligands wherein one cyclic carbon atom is substituted by

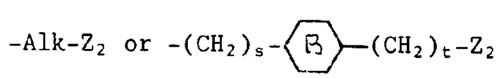

wherein ring B, s and t are as defined above, Alk is $C_{1-11}$alkylene and $Z_2$ is -NCS or $NH_2$ optionally substituted by a protecting group,

those derived from N-substituted or C-substituted macrocyclic amines including also cyclames, e.g. as disclosed in EP 304,780 A1 and in WO 89/01476-A, groups of formula IIa or IIb,

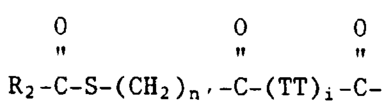 (IIa)

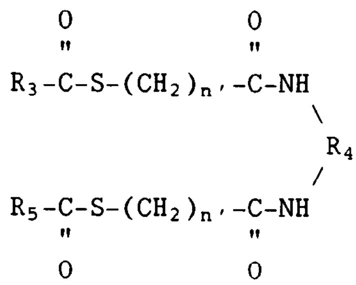

(IIb)

wherein
each of $R_2$, $R_3$ and $R_5$ independently is $C_{1-6}$alkyl, $C_{6-8}$aryl or $C_{7-9}$arylalkyl, each optionally substituted by OH, $C_{1-4}$alkoxy, COOH or $SO_3H$,

$R_4$ is 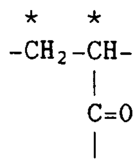 or 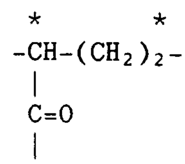

wherein the carbon atoms marked with * are attached to the imino groups,
n' is 1 or 2,
i is an integer from 2 to 6, and
TT are independently α or β amino acids linked to each other by amide bonds,
e.g. as disclosed in EP 247,866 A1
groups derived from bis-aminothiol derivatives, e.g. compounds of formula III

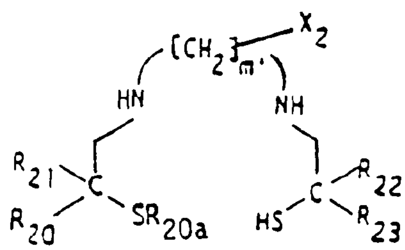

(III)

wherein
each of $R_{20}$, $R_{20a}$, $R_{21}$, $R_{22}$ and $R_{23}$ independently is hydrogen or $C_{1-4}$alkyl,
$X_2$ is either a group capable of reacting with the terminal amino group of the peptide, or a group capable of binding with the spacer group and
m' is 2 or 3,
groups derived from dithiasemicarbazone derivatives, e.g. compounds of formula IV

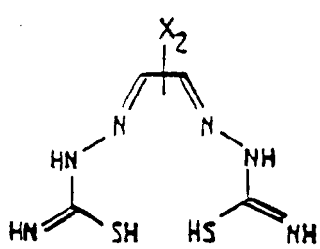

(IV)

wherein
$X_2$ is as defined above,
groups derived from propylene amine oxime derivatives, e.g. compounds of formula V

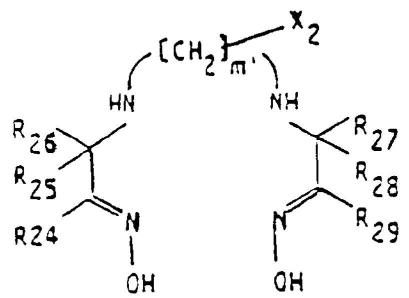

(V)

wherein
each of $R_{24}$, $R_{25}$, $R_{26}$, $R_{27}$, $R_{28}$ and $R_{29}$ independently are hydrogen or $C_{1-4}$alkyl, and
$X_2$ and m' are as defined above,
groups derived from diamide dimercaptides, e.g. compounds of formula VI

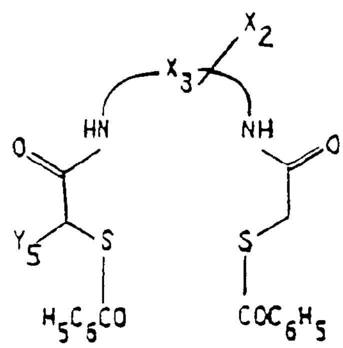

(VI)

wherein
$X_2$ is as defined above,
$X_3$ is $C_{1-4}$alkylene, $C_{1-4}$alkylene substituted by one or two $CO_2R_{30}$, by $CH_2COR_{30}$, $CONH_2$ or $CONHCH_2CO_2R_{30}$, phenylene, or phenylene substituted by $CO_2R_{30}$ wherein $R_{30}$ is $C_{1-4}$alkyl, and
$Y_5$ is hydrogen or $CO_2R_{30}$,
groups derived from porphyrins, e.g. N-benzyl-5,10,15,20-tetrakis-(4-carboxyphenyl)porphine or TPP bearing a group $X_2$ as defined above, or from Desferal (Deferoxamine).

The contents of all the above publications including the specific compounds are specifically incorporated herein by reference.

Suitable examples for $X_2$ inlcude e.g. Alk-$Z_2$ or

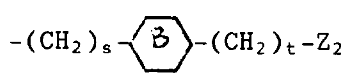 as defined above.

as defined above.

In the residue

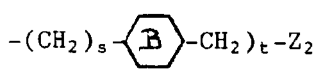,

s is preferably 1; t is preferably O; ring B is preferably phenyl; $Z_2$ is preferably -NCS or NCO.

As will be appreciated, where the chelating group present in the compounds of the invention contains vicinal carboxylic acid groups, these may also be present as anhydride functional groups.

Preferred chelating groups A are those derived from polyamino-polycarboxylic groups, e.g. those derived from EDTA, DTPA, DOTA, TETA, TETRA or TITRA optionally C-functionalised, e.g.

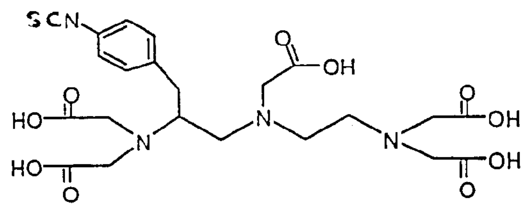

Chelating groups derived from DTPA are most preferred.

The compounds of formula I may exist e.g. in free or salt form. Salts include acid addition salts with e.g. organic acids, polymeric acids or inorganic acids, for example hydrochlorides, acetates and trifluoroacetates and salt forms obtainable with the carboxylic or sulphonic acid groups present in the chelating group, e.g. alkali metal salts such as sodium or potassium, or substituted or unsubstituted ammonium salts.

The present invention also includes a process for the production of the compounds of the invention. They may be produced by analogy to known methods.

The compounds of the invention may be produced for example as follows:

a) removing at least one protecting group which is present in a compound of formula I in protected form, or

b) linking together by an amide bond two peptide fragments each of them containing at least one amino acid in protected or unprotected form and one of them containing the chelating group and optionally a spacer group and another one containing a substituted side chain group, wherein the amide bond is in such a way that the desired amino acid sequence of formula I is obtained, and stage a) of the process is then optionally effected, or

c) linking together a chelating agent and the desired 10- or 11-substituted MSH peptide in protected or unprotected form in such a way that the chelating group is fixed on the terminal amino group of the peptide either directly or indirectly through a spacer group, and stage a) is then optionally effected, or

d) substituting the position 10 or 11 of a compound of formula $I_x$

$$A\,[X - Y_x]_n \qquad I_x$$

wherein A, X and n are as defined above, and

$Y_x$ is a residue derived from a α- or β-MSH peptide which is unsubstituted in position 10 or 11, optionally in protected form, and stage a) is then optionally effected,

and recovering the compound of formula I thus obtained in free form or in salt form.

The above reactions may be effected in analogy with known methods, e.g. as described in the following examples, in particular processes a) and c). When the chelating group is attached by an amide bond, this may be carried out analogously to the methods used for amide formation. Where desired, in these reactions, protecting groups which are suitable for use in peptides or for the desired chelating groups may be used for functional groups which do not participate in the reaction. The term protecting group may also include a polymer resin having functional groups. The peptide fragment bearing the chelating group and optionally a spacer group and used in stage b) may be prepared by reacting the peptide fragment comprising at least one amino acid in protected or unprotected form with the chelating agent. The reaction may be performed in analogy with stage c).

The chelating groups of formula IV or V may be linked to a peptide by reacting a chelating agent of formula IIaa or IIbb

$$R_2-\overset{O}{\overset{\|}{C}}-S-(CH_2)_{n'}-\overset{O}{\overset{\|}{C}}-(TT)_i-\overset{O}{\overset{\|}{C}}-X_4 \qquad \text{(IIaa)}$$

```
    O            O
    "            "
R3-C-S-(CH2)n'-C-NH-CH2     O
                       \    "
                        CH-C-X4          (IIbb)
                       /
R5-C-S-(CH2)n'-C-NH
   "             "
   O             O
```

wherein $X_4$ is an activating group capable of forming an amide bond with the N-amino group of the peptide. The reaction may be performed as disclosed in EP 247,866 A1.

The chelating agent used in process step c) may be known or prepared in analogy with known procedures. The compound used is such that it allows the introduction of the desired chelating group on the MSH peptide, e.g. a polyaminopolycarboxylic acid as disclosed, a salt or anhydride thereof.

According to a preferred embodiement of process step c), for the production of a compound of formula I wherein n is 1, the chelating group may be activated, e.g. converted into the corresponding hydrazide by reaction with e.g. hydrazine hydrate. The hydrazide chelating agent may then be reacted with the peptide in analogy with known methods, e.g. via azide coupling after conversion into the corresponding azide.

In the above process steps b) and c), when it is desired to produce a peptide in which the chelating group is attached by means of a spacer group to the amino acid of the peptide, the spacer group may be present on the corresponding amino-acids, peptide fragments or peptides used as starting material, or attached to the chelating group. The spacer group may be attached in analogy with known methods, by reacting the amino-acid, peptide fragment, peptide or chelating agent with a spacer-yielding compound, e.g. a compound of formula HO-CO-R-COOH or $H_2N$-R-COOH or a functional reactive derivative thereof.

The amino acid, peptide fragment or peptide comprising a substituted side chain group, used as starting material in process step b) may be prepared according to known methods. Process step d) may be effected in accordance with known methods. For example the substitution of a side chain amino group with a radical of formula (b) may be effected by reacting an amino-acid, peptide fragment or peptide having an unsubstituted side chain amino group with a compound of formula VII

$$HOCH_2-(CHOH)_f-\left(\begin{array}{c} Y_a \\ | \\ C \\ | \\ Y_b \end{array}\right)_g-CHO \qquad (VII)$$

wherein f, g, $Y_a$ and $Y_b$ are as defined above under reductive amination conditions, e.g. as disclosed in Example 1a).

The compounds of formula I in free form or in the form of pharmaceutically acceptable salts are valuable compounds.

According to a further embodiment, the compounds of formula I can be complexed with a detectable element.

Accordingly, the present invention also provides the compounds of formula I as defined above which are complexed with a detectable element (hereinafter referred to as CHELATES OF THE INVENTION), in free form or in salt form, their preparation and their use for in vivo diagnostic and therapeutic treatment.

By detectable element is meant any element, preferably a metal ion which exhibits a property detectable in therapeutic or in vivo diagnostic techniques, e.g. a metal ion which emits a detectable radiation or a metal ion which is capable of influencing NMR relaxation properties.

Suitable detectable metal ions include for example heavy elements or rare earth ions, e.g. as used in CAT scanning (Computer axial tomography), paramagnetic ions, e.g. $Gd^{3+}$, $Fe^{3+}$, $Mn^{2+}$ and $Cr^{2+}$, fluorescent metal ions, e.g. $Eu^{3+}$, and radionuclides, e.g. $\gamma$-emitting radionuclides, $\beta$-emitting radionuclides, $\alpha$-emitting radionuclides, positron-emitting radionuclides e.g. $^{68}Ga$, and nuclides with electron capture and resulting Auger electron cascades.

Suitable $\gamma$-emitting radionuclides include those which are useful in diagnostic techniques. The $\gamma$-emitting radionuclides advantageously have a half-live of from 1 hour to 40 days, preferably from 5 hours to 4 days. Examples are radionuclides derived from Gallium, Indium, Technetium, Ytterbium, Rhenium and Thallium e.g. $^{67}Ga$, $^{111}In$, $^{99m}Tc$, $^{169}Yb$ and $^{186}Re$. Preferably the $\gamma$-radionuclide is selected depending on the metabolism of the compounds of formula I. More preferably the compounds of formula I are chelated with a $\gamma$-radionuclide having a longer half-life than the half-life of the $\alpha$-MSH peptide on the tumor.

Further radionuclides suitable for use in imaging are positron-emitting radionuclides, e.g. as mentioned above.

Suitable $\beta$-emitting radionuclides include those which are useful in therapeutic applications, for example $^{90}Y$, $^{67}Cu$, $^{186}Re$, $^{188}Re$, $^{169}Er$, $^{121}Sn$, $^{127}Te$, $^{131}I$, $^{143}Pr$, $^{198}Au$, $^{109}Pd$, $^{165}Dy$, $^{32}P$,$^{142}Pr$. The $\beta$-radionuclide advantageously have a half-life of from 2.3 hours to 14.3 days, preferably from 2.3 to 100 hrs. Preferably the $\beta$-emitting radionuclide is selected in order to have a longer half-life than the half-life of the $\alpha$-MSH peptide on the tumor.

Suitable $\alpha$-emitting radionuclides are those which are used in therapeutic treatments, e.g. $^{211}At$, $^{212}Bi$.

Nuclides with potentially useful Auger-$e^-$-cascades following electron capture are e.g. $^{51}Cr$, $^{67}Ga$, $^{71}Ge$,

$^{77}Br$, $^{97}Ru$, $^{119}Sb$, $^{123}I$, $^{129}Cs$, $^{140}Nd$, $^{165}Er$, $^{177}Ta$, $^{197}Hg$, or $^{201}Tl$.

The CHELATES may be prepared by reacting a compound of formula I in free form or in a salt form with a corresponding detectable element yielding compound, e.g. a metal salt, preferably a water-soluble salt. The reaction may be carried out by analogy with known methods, e.g. as disclosed in Perrin, Organic Ligand, Chemical Data Series 22. NY Pergamon Press (1982); in Krejcarit and Tucker, Biophys. Biochem. Res. Com. 77: 581 (1977) and in Wagner and Welch, J. Nucl. Med. 20: 428 (1979).

Preferably the complexing of the compound of formula I is effected at a pH at which it is physiologically stable. Depending on the chelating group present, the labeling efficiency may approach 100% so that purification is not required. Radionuclides such as for example Technetium-99m may be used in oxidized form, e.g. Tc-99m pertechnetate, which may be complexed under reducing conditions.

The above mentioned reactions are conveniently effected under conditions avoiding trace metal contamination. Preferably distilled de-ionized water, ultrapure reagents, chelation-grade radio-activity etc.. are used to reduce the effects of trace metal.

The CHELATES OF THE INVENTION may exist e.g. in free or salt form. Salts include acid addition salts with e.g. organic acids, polymeric acids or inorganic acids, for example hydrochlorides and acetates, and salt forms obtainable with the carboxylic acid groups present in the molecule which do not participate to the chelate formation, e.g. alkali metal salts such as sodium or potassium, or substituted or unsubstituted ammonium salts.

The CHELATES OF THE INVENTION and their pharmaceutical acceptable salts exhibit pharmaceutical activity and are therefore useful either as an imaging agent, e.g. visualisation of α-MSH receptor positive tumors and metastases when complexed with a paramagnetic, a γ-emitting metal ion or a positron-emitting radio-nuclide, or as a radiopharmaceutical for the treatment in vivo of α-MSH receptor positive tumors and metastases when complexed with a α- or β-radionuclide or a nuclide with electron capture and resulting Auger-$e^-$-cascades as indicated by standard tests.

In particular, the CHELATES OF THE INVENTION possess binding affinity for α-MSH receptors expressed or overexpressed by tumors and metastases, as indicated in standard in vitro binding assays.

The binding medium consists of the modified Eagle's medium (MEM) used for tissue culture, supplemented with 25 mM Hepes (MEM-Hepes), 0.2 % BSA and 0.3 mM 1,10-phenanthroline. The binding reaction is started by adding 0.5 ml of B 16 mouse melanoma cell suspension (0.5 - 2 x $10^7$ cells/ml) to 12 - 75 mm polystyrene tubes containing 50 μl an $^{111}In$ labelled compound of formula I and 50 μl unlabelled compound of formula I. The concentration of the labelled peptide is 200,000 cpm/ml. For equilibrium binding, the cells are incubated at 15 ° C for 3 h and resuspended by occasional gentle mixing. Triplicate 150 μl aliquots are layered on top of 150 μl silicon oil in 0.4 ml polyethylene microtubes. The oil is prepared to a density of 1013 $kgm^{-3}$ by mixing equal volumes of AR 20 and AR 200 (Wacker Chemie GmbH, Munich, FRG). After centrifugation for 1 -2 minutes at 4 ° C the tubes are cut with a scalpel through the oil layer and the radioactivities of the cell pellet and the supernatant are measured in a γ-counter. The data from competition and saturation experiments are analyzed with computer. It is observed that the CHELATES, e.g. the labelled compound of Example 2, bind to the melanoma cells.

The affinity of the CHELATES OF THE INVENTION for α-MSH receptors can also be shown by in vivo testing.

B16 melanoma tumour is initiated in mice by inguinal injection of 2 x $10^5$ B16-F1 cell, suspended in 100 μl phosphate-buffered saline, into each side of the animal. Primary tumors up to 1.5 cm diameter develop within 2 - 3 weeks. $^{111}In$ labelled compound of formula I is injected into the jugular vein. Mice are killed at 1h, 4h and 24h after injection and tumour, skin, liver, kidneys, heart, small intestine and spleen are removed. Radioactivity in each organ is measured. 4 hours after injection the tumor/background ratio is higher than after 1 hour and does not increase significantly.

Accordingly, in a series of specific or alternative embodiments, the present invention also provides:

1. A method for in vivo detection of melanomas and metastases thereof in a subject which comprises a) administering a CHELATE OF THE INVENTION to said subject and b) recording the localisation of the receptors targeted by said CHELATE. CHELATES OF THE INVENTION for use in the in vivo detection method of the invention are the CHELATES which are complexed with a γ-emitting radionuclide, a positron-emitting radionuclide or a paramagnetic metal ion, e.g. as indicated above. The CHELATES OF THE INVENTION for use as an imaging agent in method (1) may be administered parenterally, preferably intravenously, e.g. in the form of injectable solutions or suspensions, preferably in a single injection. The appropriate dosage will of course vary depending upon, for example, the compound of formula I and the type of detectable element used, e.g. the radionuclide. A suitable dose to be injected is in the range to enable imaging by photoscanning procedures known in the art. When a radiolabelled CHELATE OF THE INVENTION is used, it may advantageously be administered in a dose having a radioactivity of from 1 μCi to 50 mCi. An indicated dosage range may be of from 1 to 200 μg compound of formula I labelled with 1 μCi to 50 mCi γ-emitting radionuclide, depending on

the γ-emitting radionuclide used. For example with In, it is preferred to use a radioactivity in the lower range, whereas with Tc, it is preferred to use a radioactivity in the upper range.

The enrichment in the tumorigenic sites with the CHELATES may be followed by the corresponding imaging techniques, e.g. using nuclear medicine imaging instrumentation, for example a scanner, γ-camera , rotating γ-camera, each preferably computer assisted (SPECT; PET); MRI equipment or CAT scanning equipment.

The CHELATES OF THE INVENTION may also be useful in assisting surgeons in locating melanomas and metastases intraoperatively (Radioimmuno guided surgery).

2. A method for in vivo treatment of melanomas and metastases in a subject in need of such a treatment which comprises administering to said subject a therapeutically effective amount of a CHELATE OF THE INVENTION.

CHELATES OF THE INVENTION for use in the in vivo treatment method of the invention are the CHELATES complexed with a α- or β-radionuclide or a nuclide with electron capture and resulting Auger-$e^-$-cascades as defined above.

Dosages employed in practising the therapeutic method of the present invention will of course vary depending e.g. on the particular condition to be treated, for example the volume of the tumor, the particular CHELATE employed, for exemple the half-life of the CHELATE in the tumor, and the therapy desired. In general, the dose is calculated on the basis of radioactivity distribution to each organ and on observed target uptake. For example the CHELATE may be administered at a daily dosage range having a radioactivity of from 0.1 μCi/kg to 3mCi/kg body weight. An indicated daily dosage range is of from 1 to 200 μg compound of formula I labelled with 0.1 μCi/kg to 3 mCi/kg body weight α- or β-emitting radionuclide or a nuclide with electron capture and resulting Auger-$e^-$-cascades, conveniently administered in divided doses up to 4 times a day.

The α-, β- or Auger electrons emitting CHELATES OF THE INVENTION may be administered by any conventional route, e.g. parenterally, in particular intravenously, e.g. in the form of injectable solutions or suspensions. They may also be administered advantageously by infusion, e.g. an infusion of 30 to 60 min. Depending on the site of the tumor, they may be administered as close as possible to the tumor site, e.g. by means of a catheter. The mode of administration selected may depend on the dissociation rate of the CHELATE used and the excretion rate.

The CHELATES OF THE INVENTION may be administered in free form or in pharmaceutically acceptable form. Such salts may be prepared in conventional manner and exhibit the same order of activity as the free compounds.

The CHELATES OF THE INVENTION for use in the method of the present invention may preferably be prepared shortly before the administration to a subject, i.e. the radiolabeling with the desired detectable metal ion, particularly the desired α-, β- or γ-radionuclide, may be performed shortly before the administration.

According to a further aspect of the invention, there is provided:

i. a pharmaceutical composition comprising a compound of formula I in free or in pharmaceutically acceptable salt form, together with one or more pharmaceutically acceptable carriers or diluents therefor;

ii. a pharmaceutical composition comprising a CHELATE according to the invention in free or in pharmaceutically acceptable salt form, together with one or more pharmaceutically acceptable carriers or diluents therefor.

Such compositions may be manufactured in conventional manner, e.g. mixing of the ingredients.

A composition according to the invention may also be presented in separate package with instructions for mixing the compound of formula I with the metal ion and for the administration of the resulting CHELATE. It may also be presented in twin-pack form, that is, as a single package containing separate unit dosages of compound of formula I and the detectable metal ion with instructions for mixing them and for administration of the CHELATE. A diluent or carrier may be present in the unit dosage forms.

In a series of specific embodiments, the present invention also provides a α-MSH peptide bearing

i) in the 10- or 11-position a side chain amino group which is mono- or disubstituted; and

ii) a chelating group linked to the terminal amino group of the peptide;

the chelating group being capable of complexing a detectable element and the substitution on the side chain amino group in the 10- or 11-position being selected so that it does not significantly interfere with or impair the binding affinity of the peptide to the α-MSH receptors.

In the following examples, all temperatures are in ° C and $[\alpha]_D^{20}$-values uncorrected. The following abbreviations are employed:

AcOH acetic acid
DMF dimethylformamide
Boc tert.-butoxycarbonyl
TFA trifluoroacetic acid

```
            HOOCH2C                      CH2COOH
                   \                     /
DTPA                N-CH2CH2-N-CH2CH2-N
                   /         |           \
            HOOCH2C        CH2COOH      CH2CO-
```

or a corresponding divalent radical for examples 5b), 7 and 8
Bum tert.-butoxymethyl
Pmc pentamethylchromansulfonyl

**EXAMPLE 1: DTPA-Nle-Asp-His-DPhe-Arg-Trp-Lys($N^\varepsilon$-bis-2,3-dihydroxypropyl)-$NH_2$**

40 mg (0.1 mM) DTPA-$NHNH_2$ are suspended in 2 ml DMF and reacted with 40 µl 3 N HCl in diethyl ether. 13.5 µl (0.105 mM) tert.-butyl nitrite (90 %) is then added at - 10 ° dropwise to the resulting solution and the mixture is stirred for 30 minutes at - 10 °. 90 mg (0.075 mM) H-Nle-Asp-His-DPhe-Arg-Trp-Lys($N^\varepsilon$-bis-2,3-dihydroxypropyl)-$NH_2$ are dissolved in 2 ml DMF and 40 µl Huenig's base are added thereto. The resulting solution is added to the DTPA-azide solution. The pH is adjusted to 8 - 9 by the addition of Huenig's base and the mixture is further stirred at 0 °. The reaction is followed by thin layer chromatography. When the reaction is complete, the mixture is evaporated, yielding the title compound which is purified by HPLC.
$MH^+$ = 1523
$[\alpha]_D^{20}$ = - 20.0 ° (c = 0.22 in 95 % AcOH)

The starting material is prepared as follows:

a) Fmoc Nle-Asp-His-DPhe-Arg-Trp-Lys($N^\varepsilon$-bis-2,3-diisopropylidene-2,3-dihydroxy-propyl)-$NH_2$

230 mg (0.19 mM) Fmoc Nle-Asp-His-DPhe-Arg-Trp-Lys-$NH_2$ are dissolved in 30 ml of a 2 : 1 $CH_3OH$/water mixture. 120 mg (1.9 mM) sodium cyanoborohydride and thereafter 250 mg (1.9 mM) 2,3-diisopropylidene glycerine aldehyde are added and the whole mixture is adjusted to pH 5.5 with 1 N HCl. After 18 hours, the mixture is concentrated and then azeotropically evaporated three times with toluene. The residue is chromatographied on a silica gel column using a 8 : 2 : 0.125 : 0.125 mixture of chloroform/methanol/AcOH/water.
$MH^+$ = 1430
$[\alpha]_D^{20}$ = - 22.8 ° (c = 0.30 in 95 % AcOH)

b) H-Nle-Asp-His-DPhe-Arg-Trp-Lys($N\varepsilon$-bis-2,3-dihydroxypropyl)-$NH_2$

140 mg of the peptide obtained under a) above are stirred in 10 ml of 1 : 4 piperidin/DMF at room temperature for 15 minutes. The mixture is then evaporated and immediately thereafter dissolved in 10 ml acetonitrile. 0.24 ml 35 % $HBF_4$ are added and the mixture is stirred at room temperature for 30 minutes. 0.5 ml triethylamine are then added and the mixture is evaporated. The residue is chromatographied on a silica gel column using a 7 : 5 : 2 : 2 mixture of chloroform/methanol/AcOH/water.
$MH^+$ = 1149
$[\alpha]_D^{20}$ = - 17.5 ° (c = 0.2 in 95 % AcOH)

**EXAMPLE 2: $^{111}$In labelled DTPA-Nle-Asp-His-DPhe-Arg-Trp-Lys($N^\varepsilon$-bis-2,3-dihydroxypropyl)-$NH_2$**

1 mg DTPA-Nle-Asp-His-DPhe-Arg-Trp-Lys($N^\varepsilon$-bis-2,3-dihydroxypropyl)-$NH_2$ is dissolved in 5 ml 0.01M acetic acid. The resulting solution is passed through a 0.22µ Millex-GV filter and dispensed in 0.1 ml portions and stored at -20°C. $^{111}InCl_3$ (Amersham, 1 mCi/100 µl) is prediluted in an equal volume of 0.5M sodium acetate and labeling is carried out by mixing the ligand with the $InCl_3$ solution and gentle homogenisation at room temperature.

HEPES buffer, pH 7.4, and 1% BSA are then added.

**EXAMPLE 3: $^{90}$Y labelled DTPA-Nle-Asp-His-DPhe-Arg-Trp-Lys($N^\varepsilon$-bis-2,3-dihydroxypropyl)-$NH_2$**

$^{90}$Y is obtained from a $^{90}$Sr-$^{90}$Y radionuclide generator. The construction of the generator, its elution and the conversion of the [$^{90}$Y]EDTA to the acetate complex are performed in accordance with the method disclosed by M.Chinol and D.J. Hnatowich in J. Nucl. Med. 28, 1465-1470 (1987). 1 mg of DTPA-Nle-Asp-His-DPhe-Arg-Trp-Lys($N^\varepsilon$-bis-2,3-dihydroxypropyl)-$NH_2$ dissolved in 5ml 0.01M acetic acid is allowed to warm to room temperature and 1.0 mCi of $^{90}$Y in 50 µl sterile 0.5M acetate is added. The mixture is then left undisturbed for 30

min to 1 hr to maximize chelation.

**EXAMPLE 4: Acetyl-Nle-Asp-His-DPhe-Arg-Trp-Lys($N^{\varepsilon}$-bis-2,3-dihydroxypropyl)-$NH_2$**

50 mg (0.044 mM) of the compound of Example 1b) are treated with 24 µl (0.24 mM) acetic anhydride and 0.24 ml triethylamin in a 1 : 1 dioxan/water mixture. After 16 hours the mixture is neutralized with 0.25 ml AcOH, evaporated and the residue is chromatographied on a silica gel column using a 7 : 4 : 1 : 1 mixture of chloroform/methanol/AcOH/water. The fractions containing the title compound are collected, purified by HPLC and desalted.

$[\alpha]_D^{20}$ = - 32.9 ° (c = 0.24 in 95 % AcOH)

This compound may be used as a reference in the binding assays.

**EXAMPLE 5:**

a) **DTPA-Nle-Asp-His-DPhe-Arg-Trp-Lys($N^{\varepsilon}$-2,3-dihydroxypropyl)-$NH_2$**

b) **DTPA (Nle-Asp-His-DPhe-Arg-Trp-Lys($N^{\varepsilon}$-2,3-dihydroxypropyl)-$NH_2$)$_2$**

1g of Fmoc-Nle-Asp(OBu)-His(Bum)-DPhe-Arg(Pmc)-Trp-Lys(Boc,2,3-iso-propylidene-2,3-dihydroxypropyl)-$NH_2$ are stirred in 10 ml of piperidine/DMF at room temperature for about 15 minutes. After removing the solvent the residue is purified by flash chromatography using silica gel as stationary phase and ethyl acetate and ethyl acetate/methanol 2/1 as eluent.

500 mg of the end product described above are dissolved in 35 ml of dioxane/water. After addition of 150 mg of $NaHCO_3$ and 150 mg of DTPA dianhydride the reaction mixture is kept at room temperature for 30 minutes. After removal of dioxane and adjusting the pH to 6 with IN HCl, the bis-substituted DTPA derivative precipitates, is filtered off and dried. The mothor liquor is lyophilized and contains most of the mono-DTPA derivative. Both solids are treated with 5 ml TFA/$H_2O$ for 90 minutes each and after addition of diethylether the compounds precipitate, are filtered off and dried.

Purification on silica gel with chloroform/methanol/glacial acetic acid 50/80/30 and desalting on RP-column (C-18) give a) in pure form (mono-substituted) $MH^+$: 1450.6 and b)in pure form (di-substituted) $MH^+$: 2505.8

**EXAMPLE 6: $^{111}$In labeled DTPA-Nle-Asp-His-DPhe-Arg-Trp-Lys($N^{\varepsilon}$-2,3-dihydroxypropyl)-$NH_2$**

By following the procedure of Example 2, the title compound is obtained.

**EXAMPLE 7: $^{111}$In labeled DTPA (Nle-Asp-His-DPhe-Arg-Trp-Lys($N^{\varepsilon}$-2,3-dihydroxypropyl)-$NH_2$)$_2$**

By following the procedure of Example 2, the title compound is obtained.

**EXAMPLE 8: 111In labeled DTPA (Nle-Asp-His-DPhe-Arg-Trp-Lys($N^{\varepsilon}$-bis-2,3-dihydroxypropyl)-$NH_2$)$_2$**

By following the procedures of Examples 5 and 2, the title compound is obtained.

## Claims

**1.** A compound of formula I

$$A[X - Y]_n \qquad (I)$$

wherein
A is a chelating group capable of complexing a detectable element,
n is 1 or 2
X is a direct bond or a spacer group, and
Y is a residue derived from a α- or β-MSH peptide bearing in the 10- or 11-position a substituted side chain group
A-X being linked to the terminal amino group of the peptide residue Y and the substitution on the side chain group in the 10- or 11-position being selected so that it does not significantly interfere with or impair the binding affinity of the peptide to the MSH receptors,

in free form or in the form of a salt or complex.

2. A compound according to claim 1 wherein Y bears in position 10 or 11 a mono- or disubstituted side chain amino group.

3. A compound of formula IA

$$A[X - Y_1]_n \qquad (I)$$

wherein
A is a chelating group capable of complexing a detectable element,
n is 1 or 2
X is a direct bond or a spacer group, and
$Y_1$ is (Nle$^4$, D-Phe$^7$]$\alpha$-MSH comprising in position 10 or 11 a mono- or disubstituted side chain amino group, A-X being linked to the terminal amino group of the peptide residue $Y_1$ and the substitution on the side chain amino group in the 10- or 11-position being selected so that it does not significantly interfere with or impair the binding affinity of the peptide to the MSH receptors,
in free form or in the form of a salt or complex.

4. A compound according to any one of the preceding claims, wherein the side chain group in position 10 or 11 is a side chain amino group mono- or disubstituted by a radical of formula (b)

$$HOCH_2-(CHOH)_f-(\overset{\overset{Y_a}{|}}{\underset{\underset{Y_b}{|}}{C}})_g-CH_2- \qquad (b)$$

wherein one of $Y_a$ and $Y_b$ is hydrogen and the other is hydroxy or both are hydrogen, and
each of f and g independently is 0 or 1.

5. A compound of formula I

$$A[X - Y]_n \qquad (I)$$

wherein
A is a chelating group capable of complexing a detectable element,
n is 1 or 2
X is a direct bond or a spacer group, and
Y is [Nle$^4$, Asp$^5$, DPhe$^7$, (N$^\varepsilon$-bis-2,3-dihydroxypropyl-Lys)$^{10}$]$\alpha$-MSH$_{(4-10)}$NH$_2$ in free form or in the form of a salt or complex.

6. A compound according to any one of the preceding claims, wherein A is a radical derived from ethylene diaminetetraacetic acid (EDTA), diethylene triamine pentaacetic acid (DTPA), N-hydroxyethyl-N,N′,N′-ethylene diaminetriacetic acid (HEDTA), ethylene glycol-0,0′-bis(2-aminoethyl)-N,N,N′,N′-tetraacetic acid (EGTA), N,N′-bis(hydroxybenzyl)ethylenediamine-N,N′-diacetic acid (HBED), triethylenetetramine hexaacetic acid (TTHA), derived from substituted EDTA or DTPA, derived from 1,4,7,10-tetra-azacyclododecane-N,N′,N″,N‴-tetraacetic acid (DOTA), 1,4,8,11-tetraazacyclotetradecane- N,N′,N″,N‴-tetraacetic acid (TETA), 1,4,7,-10-tetraazacyclotridecane-1,4,7,10-tetraacetic acid (TITRA), 1,4,8,11-tetraazacyclotetradecane (TETRA) or their C-functionalised derivatives thereof,
those derived from a group of formula IIa or IIb,

$$R_2-\overset{\overset{O}{\|}}{C}-S-(CH_2)_{n'}-\overset{\overset{O}{\|}}{C}-(TT)_i-\overset{\overset{O}{\|}}{C}- \qquad (IIa)$$

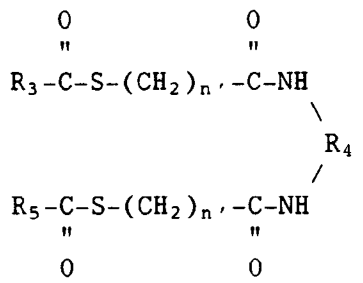

(IIb)

wherein
each of $R_2$, $R_3$ and $R_5$ independently is $C_{1-6}$alkyl, $C_{6-8}$aryl or $C_{7-9}$arylalkyl, each optionally substituted by OH, $C_{1-4}$alkoxy, COOH or $SO_3H$,
n' is 1 or 2,
i is an integer from 2 to 6, and
TT are independently α or β amino acids linked to each other by amide bonds,
a compound of formula III

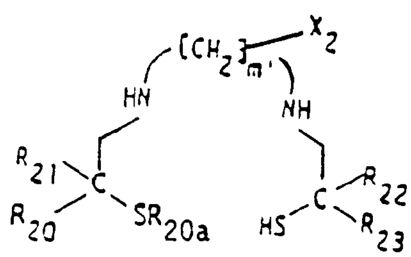

(III)

wherein
each of $R_{20}$, $R_{20a}$, $R_{21}$, $R_{22}$ and $R_{23}$ independently is hydrogen or $C_{1-4}$alkyl,
$X_2$ is either a group capable of reacting with the terminal amino group of the peptide, or a group capable of binding with the spacer group and
m' is 2 or 3,
a group derived from dithiasemicarbazone derivatives of formula IV

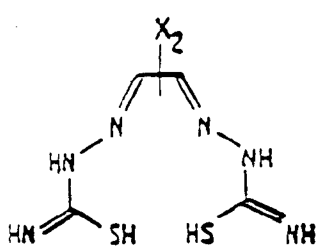

(IV)

wherein
$X_2$ is as defined above,
a group derived from propylene amine oxime derivatives of formula V

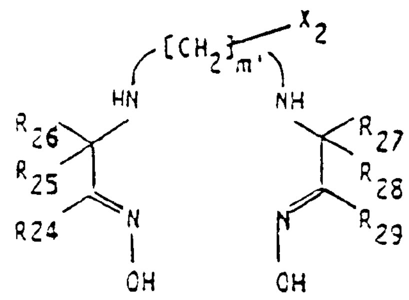

(V)

wherein
each of $R_{24}$, $R_{25}$, $R_{26}$, $R_{27}$, $R_{28}$ and $R_{29}$ independently are hydrogen or $C_{1-4}$alkyl, and
$X_2$ and m' are as defined above,
a group derived from diamide dimercaptides, e.g. compounds of formula VI

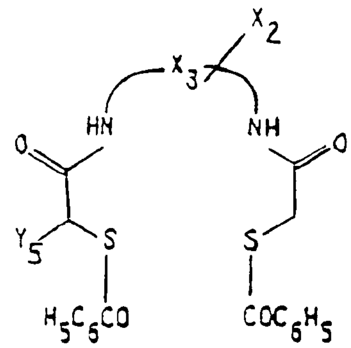

(VI)

wherein
$X_2$ is as defined above,
$X_3$ is $C_{1-4}$alkylene, $C_{1-4}$alkylene substituted by one or two $CO_2R_{30}$, by $CH_2COR_{30}$, $CONH_2$ or $CONHCH_2CO_2R_{30}$, phenylene, or phenylene substituted by $CO_2R_{30}$ wherein $R_{30}$ is $C_{1-4}$alkyl, and
$Y_5$ is hydrogen or $CO_2R_{30}$,
a group derived from porphyrins or from Deferoxamine.

7. A compound according to any one of claims 1 to 5 wherein A is a radical derived from diethylene triamine pentaacetic acid (DTPA).

8. A process for the production of a compound of formula I as defined in claim 1, comprising
a) removing at least one protecting group which is present in a compound of formula I in protected form, or
b) linking together by an amide bond two peptide fragments each of them containing at least one amino acid in protected or unprotected form and one of them containing the chelating group and optionally a spacer group and another one containing a substituted side chain group, wherein the amide bond is in such a way that the desired amino acid sequence of formula I is obtained, and stage a) of the process is then optionally effected, or
c) linking together a chelating agent and the desired 10- or 11-substituted MSH peptide in protected or unprotected form in such a way that the chelating group is fixed on the terminal amino group of the peptide either directly or indirectly through a spacer group, and stage a) is then optionally effected, or
d) substituting the position 10 or 11 of a compound of formula $I_x$

$$A[X - Y_x]_n \qquad I_x$$

wherein A, X and n are as defined in claim 1, and
$Y_x$ is a residue derived from a α- or β-MSH peptide which is unsubstituted in position 10 or 11, optionally in protected form, and stage a) is then optionally effected,
and recovering the compound of formula I thus obtained in free form or in salt form.

9. A compound according to any one of claims 1 to 7, complexed with a detectable element.

**10.** A compound according to any one of claims 1 to 7, complexed with a detectable element, for use as imaging agent or therapeutic agent.

**11.** A pharmaceutical composition comprising a compound according to any one of claims 1 to 7 in free or in pharmaceutically acceptable salt form, optionally complexed with a detectable element, together with one or more pharmaceutically acceptable carriers or diluents therefor.

**12.** A package containing unit dosages of a peptide as defined in any one of claim 1 to 7 and of a detectable element with instructions for mixing them and for use as imaging agent or therapeutic agent.

**Claims for the following Contracting State : ES**

**1.** A process for the production of a compound of formula I

$$A[X - Y]_n \qquad (I)$$

wherein
A is a chelating group capable of complexing a detectable element,
n is 1 or 2
X is a direct bond or a spacer group, and
Y is a residue derived from a α- or β-MSH peptide bearing in the 10- or 11-position a substituted side chain group
A-X being linked to the terminal amino group of the peptide residue Y and the substitution on the side chain group in the 10- or 11-position being selected so that it does not significantly interfere with or impair the binding affinity of the peptide to the MSH receptors in free form or in the form of a salt or complex,
which process comprises

a) removing at least one protecting group which is present in a compound of formula I in protected form, or

b) linking together by an amide bond two peptide fragments each of them containing at least one amino acid in protected or unprotected form and one of them containing the chelating group and optionally a spacer group and another one containing a substituted side chain group, wherein the amide bond is in such a way that the desired amino acid sequence of formula I is obtained, and stage a) of the process is then optionally effected, or

c) linking together a chelating agent and the desired 10- or 11-substituted MSH peptide in protected or unprotected form in such a way that the chelating group is fixed on the terminal amino group of the peptide either directly or indirectly through a spacer group, and stage a) is then optionally effected

d) substituting the position 10 or 11 of a compound of formula $I_x$

$$A\ [X - Y_x]_n$$

wherein A, X and n are as defined in claim 1, and
$Y_x$ is a residue derived from a α- or β-MSH peptide which is unsubstituted in position 10 or 11, optionally in protected form, and stage a) is then optionally effected,

and recovering the compound of formula I thus obtained in free form or in salt form.

**2.** A process according to claim 1, for the production of a compound of formula I wherein the substituted side chain group in position 10 or 11 is a side chain amino group mono- or disubstituted by a radical of formula (b)

$$HOCH_2-(CHOH)_f-\underset{\displaystyle Y_b}{\overset{\displaystyle Y_a}{\underset{|}{\overset{|}{(C)}}}}{}_g-CH_2- \qquad (b)$$

wherein one of $Y_a$ and $Y_b$ is hydrogen and the other is hydroxy or both are hydrogen, and each of f and g independently is 0 or 1.
which process comprises reacting a MSH peptide unsubstituted in position 10 or 11, in protected or unprotected form, with a compound of formula VII

```
                    Ya
                    |
HOCH2-(CHOH)f-(C)g-CHO                    (VII)
                    |
                    Yb
```

wherein f, g, $Y_a$ and $Y_b$ are as defined above, under reductive amination conditions.

3. A process according to claim 1 or 2, for the production of a compound of formula I wherein X is a spacer group, comprising reacting in process steps b) or c), an amino-acid, peptide fragment or MSH peptide comprising a spacer group X) attached to the terminal amino group.

4. A process according to claim 1 or 2, for the production of a compound of formula I wherein X is a spacer group, comprising reacting in process steps b) or c), a chelating agent bearing the spacer group X.

5. A process according to claim 1, for the production of a compound of formula I wherein Y is (Nle$^4$, D-Phe$^7$] α-MSH comprising a mono or disubstituted side chain amino group in position 10 or 11.

6. A process according to claim 1 for the production of a compound of formula I wherein Y is (Nle$^4$, Asp$^5$, D-Phe$^7$, (N′-bis 2,3-dihydroxypropyl-Lys)$^{10}$] α-MSH$_{(4-10)}$ NH$_2$.

7. A process according to any one of the preceding claims, wherein the chelating agent is ethylene diaminetetraacetic acid (EDTA), diethylene triamine pentaacetic acid (DTPA), N-hydroxyethyl-N,N′,N′-ethylene diaminetriacetic acid (HEDTA), ethylene glycol-0,0′-bis(2-aminoethyl)-N,N,N′,N′-tetraacetic acid (EGTA), N,N′-bis(hydroxybenzyl)ethylenediamine-N,N′-diacetic acid (HBED), triethylenetetramine hexaacetic acid (TTHA), derived from substituted EDTA or DTPA, derived from 1,4,7,10-tetra-azacyclododecane-N,N′,N″,N‴-tetraacetic acid (DOTA), 1,4,8,11-tetraazacyclotetradecane-N,N′,N″,N‴-tetraacetic acid (TETA), 1,4,7,10-tetraazacyclotridecane-1,4,7,10-tetraacetic acid (TITRA), 1,4,8,11-tetraazacyclotetradecane (TETRA) or their C-functionalised derivatives thereof, a compound of formula IIaa or IIbb

```
   O              O       O
   "              "       "
R2-C-S-(CH2)n'-C-(TT)i-C-X4                    (IIaa)
```

```
   O              O
   "              "
R3-C-S-(CH2)n'-C-NH-CH2       O
                        \     "
                         CH-C-X4                (IIbb)
                        /
   R5-C-S-(CH2)n'-C-NH
      "           "
      O           O
```

wherein
each of $R_2$, $R_3$ and $R_5$ independently is $C_{1-6}$alkyl, $C_{6-8}$aryl or $C_{7-9}$arylalkyl, each optionally substituted by OH, $C_{1-4}$alkoxy, COOH or $SO_3H$,
n′ is 1 or 2,
i is an integer from 2 to 6, and
TT are independently α or β amino acids linked to each other by amide bonds,
a compound of formula III

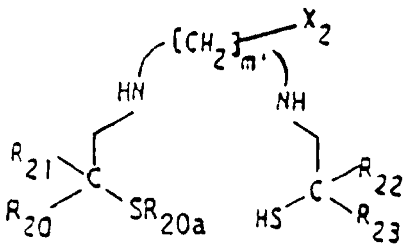

(III)

wherein
each of $R_{20}$, $R_{20a}$, $R_{21}$, $R_{22}$ and $R_{23}$ independently is hydrogen or $C_{1-4}$alkyl,
$X_2$ is either a group capable of reacting with the terminal amino group of the peptide, or a group capable of binding with the spacer group and
m' is 2 or 3,
a compound of formula IV

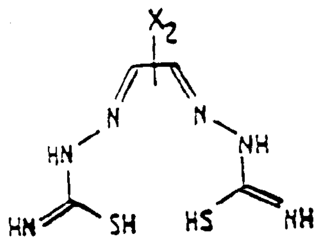

(IV)

wherein
$X_2$ is as defined above,
a compound of formula V

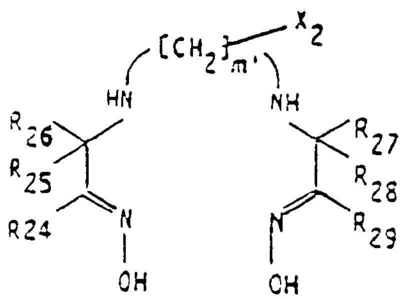

(V)

wherein
each of $R_{24}$, $R_{25}$, $R_{26}$, $R_{27}$, $R_{28}$ and $R_{29}$ independently are hydrogen or $C_{1-4}$alkyl, and
$X_2$ and m' are as defined above,
a compound of formula VI

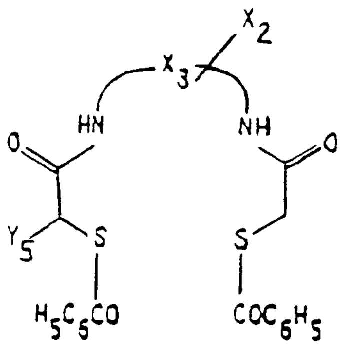

(VI)

wherein
$X_2$ is as defined above,
$X_3$ is $C_{1-4}$alkylene, $C_{1-4}$alkylene substituted by one or two $CO_2R_{30}$, by $CH_2COR_{30}$, $CONH_2$ or $CONHCH_2CO_2R_{30}$, phenylene, or phenylene substituted by $CO_2R_{30}$ wherein $R_{30}$ is $C_{1-4}$alkyl, and
$Y_5$ is hydrogen or $CO_2R_{30}$,
a porphyrin or Deferoxamine.

**8.** A process according to any one of the preceding claims, wherein the chelating agent is DTPA.

**9.** A process for the production of a compound of formula I as defined in claim 1, complexed with a detectable element, comprising reacting a compound of formula I in free form as in a salt form with a detectable element yielding compound.

**10.** A process according to claim 9, in which the detectable element yielding compound is an In, Y, Re or Tc salt.